# EUROPÄISCHE PATENTANMELDUNG

(11) **EP 2 145 669 A2**
(43) Veröffentlichungstag der Anmeldung: **20.01.2010**
(21) Anmeldenummer: 09002426.6
(22) Anmeldetag: 20.02.2009
(51) Int. Cl.: B01D 53/30, B01D 53/64

(54) **Verfahren und Vorrichtung zur Abscheidung von Quecksilber aus Abgasen**

(30) Priorität: 20.02.2008 DE 102008010195
(71) Anmelder: HIM GmbH, 64584 Biebesheim (DE)
(72) Erfinder: Artmann, Siegfried, Dr., 64625 Bensheim (DE); Zieger, Wolfgang, 68794 Oberhausen-Rheinhausen (DE); Blum, Marcus, 64584 Biebesheim (DE)
(74) Vertreter: Katscher Habermann Patentanwälte

(57) **Zusammenfassung**

Bei einem Verfahren zur konzentrationsabhängigen Steuerung der Abscheideleistung von Reinigungsstufen wird für die Bestimmung einer Quecksilberbelastung eines Abgasstroms (2) einer Abfallverbrennungsanlage ein Messgasstrom (6) aus einem noch nicht gereinigtem Abgasstrom (2) abgezweigt und in einem vorgebbaren Verhältnis mit einem reduktiven Medium vermischt und der vermischte Messgasstrom (6) anschließend einer Messvorrichtung (8) zugeführt, mittels derer eine Bestimmung der Quecksilberkonzentration erfolgt. Bei Überschreiten eines voreingestellten Konzentrationswertes von Gesamtquecksilber und metallischem Quecksilber erfolgt eine Aktivierung zusätzlicher Reinigungsstufen und je nach Vorgabe eine Regelung der Reinigungsleistung der Reinigungsstufen. Der Messgasstrom (6) wird durch beheizbare Zuführungsleitungen der Messvorrichtung (8) zugeführt. In zeitlichen Abständen wird ein Testgasstrom (10) mit metallischem Quecksilber der Messvorrichtung (8) zugeführt. Der Testgasstrom (10) wird weitgehend durch dieselben Zuführungsleitungen wie der Messgasstrom (6) den Messvorrichtungen (8) zugeführt. Der Testgasstrom (10) wird durch Kapillardiffusion eines vorgegebenen Quecksilber-II-Chloridgasstroms und Zuführung eines befeuchteten CO-Gasstromes in einen vorgegebenen Trägergasstrom (19) erzeugt.

## Beschreibung

Verfahren und Vorrichtung zur Abscheidung von Quecksilber aus Abgasen.

Die Erfindung betrifft ein Verfahren zur Abscheidung von Quecksilber aus Abgasen von Verbrennungsanlagen, insbesondere Abfallverbrennungsanlagen, wobei einem Abgasstrom geeignete Hilfsstoffe zur Abscheidung des Quecksilbers zugeführt werden.

Das bei der Abfallverbrennung entstehende gasförmige Verbrennungsprodukt wird auch als Rauchgas bezeichnet und enthält regelmäßig verschiedene Gase, Aerosole sowie Festkörperpartikel, die je nach Art des verbrannten Abfalls umweltbelastende, umweltgefährdende oder auch giftige Bestandteile enthalten können.

Bevor das bei der Abfallverbrennung entstehende Rauchgas in die Atmosphäre abgegeben werden kann, muss deshalb eine oftmals aufwändige Reinigung des Rauchgases vorgenommen werden. Zu diesem Zweck wird das aus einer Brennkammer bei der Abfallverbrennung austretende Rauchgas anschließend in einem Abgasstrom mehreren verschiedenen Reinigungsstufen zugeführt, bevor der gereinigte Abgasstrom in die Atmosphäre abgegeben werden kann. In den einzelnen Reinigungsstufen werden gezielt verschiedene gas-, aerosol- oder partikelförmige Verunreinigungen dem Abgasstrom entzogen. Dabei kommen regelmäßig völlig unterschiedliche Reinigungsverfahren zum Einsatz, wie beispielsweise Verfahren, die auf Fällungs- oder Redoxprozessen, Absorption oder Adsorption sowie auf mechanischer oder elektrostatischer Filtration beruhen.

Das unmittelbar nach der Brennkammer noch unbehandelte Rauchgas, das Rohgas, wird dabei auf dem Weg durch die Reinigungsstufen zunehmend von Verunreinigungen und Schadstoffbelastungen gereinigt, so dass nach Abschluss des Reinigungsprozesses ein weitgehend umweltneutrales Reingas in die Atmosphäre abgegeben wird.

Es bestehen gesetzliche Vorschriften, welche die maximal zulässigen Emissionen verschiedener Schadstoffe regeln, die bei der Verbrennung beispielsweise von Abfall entstehen und zeitweise je nach Abfallzusammensetzung in hoher Konzentration in dem noch nicht gereinigten Rauchgasstrom enthalten sein können. Die Einhaltung dieser Grenzwerte wird üblicherweise durch kontinuierliche Konzentrationsmessungen der relevanten Schadstoffe überwacht und dokumentiert. Diese Konzentrationsmessungen finden unmittelbar vor dem Austritt des gereinigten Abgasstroms in die Atmosphäre statt. Werden die vorgegebenen Grenzwerte überschritten, so können verschiedene Sanktionen vorgesehen sein und eine zumindest zeitweise Stilllegung der Abfallverbrennungsanlage zur Folge haben. Die verschiedenen Reinigungsstufen sind deshalb so konzipiert und ausgelegt, dass eine ungewollte Überschreitung der Grenzwerte bei Einhaltung der Annahmebedingungen der Verbrennungsanlagen möglichst ausgeschlossen werden kann.

Bei der Verbrennung von Abfall ist eine vollständige Kontrolle vor der Verbrennung insbesondere bei Haus- und Sondermüll nicht möglich. Es kann deshalb nicht zuverlässig ausgeschlossen, bzw. vermieden werden, dass bei der Verbrennung von beispielsweise nicht vollständig oder unzutreffend deklariertem Abfall unvorhergesehene Schadstoffbelastungen im Rauchgas auftreten, die in den nachfolgenden Reinigungsstufen nicht mehr vollständig dem Abgasstrom entzogen werden können. Eine Überschreitung der Grenzwerte ist in diesen Fällen nahezu unvermeidlich.

Erfahrungsgemäß treten jedoch relevante Überschreitungen der zulässigen Grenzwerte nur an wenigen Tagen im Jahr auf. Um eine vollständige Reinigung des Rauchgases auch an diesen Tagen gewährleisten zu können, müssten die einzelnen Reinigungsstufen so ausgelegt und kontinuierlich betrieben werden, dass auch die lediglich kurzzeitig auftretenden, übermäßig hohen Schadstoffbelastungen vollständig aus dem Abgasstrom entfernt werden können. Die einzelnen Reinigungsverfahren erfordern allerdings oftmals den Einsatz umweltbelastender oder giftiger Reinigungsmittel und Hilfsstoffe, so dass eine hohe Reinigungsleistung einen entsprechend hohen Verbrauch derartiger Reinigungsmittel und Hilfsstoffe bedingt und dadurch ebenfalls die Umwelt belastet. Zudem ist eine hohe Reinigungsleistung mit entsprechend hohen Betriebskosten verbunden.

In besonderem Maße tritt diese Problematik der massiven Überschreitung von Grenzwerten bei einer Belastung des Abgasstroms mit Quecksilber auf. Werden einzelne quecksilberhaltige Abfallgebinde, Flüssigkeiten oder Feststoffe verbrannt, so steigt die Quecksilberbelastung des Abfallstroms auf ein Vielfaches der üblichen Werte. Die Abscheidung von Quecksilber aus dem Abgasstrom ist jedoch mit einem erheblichen Aufwand verbunden, der unter anderem vom Oxidationszustand des Quecksilbers im Abgasstrom abhängt. Zudem kann sich das Quecksilber in den verschiedenen Reinigungsstufen zeitweilig ablagern und über einen längeren Zeitraum wieder von dem Abgasstrom aufgenommen werden, so dass auch einige Tage nach dem Verbrennen von quecksilberhaltigen Abfällen ein nicht vollständig von Quecksilber gereinigter Abgasstrom austreten kann, dessen verbleibende Quecksilberbelastung gegebenenfalls noch über den vorgegebenen Grenzwerten liegt.

Die Überschreitung der Grenzwerte für die Quecksilberbelastung sowie eine Ablagerung hoher Quecksilberkonzentrationen in den einzelnen Reinigungsstufen kann derzeit nicht gezielt vermieden werden, da eine Messung der Quecksilberbelastung mit den handelsüblichen Messvorrichtungen lediglich im Reingasbereich oder zumindest nach mindestens einer geeigneten Reinigungsstufe möglich ist.

Es sind verschiedene Verfahren und Vorrichtungen zum Messen von Quecksilberkonzentrationen bekannt. In der Druckschrift DE 10 2006 031 040 A1 wird beispielsweise ein Verfahren und eine Vorrichtung zum Messen von Quecksilber in Gasen, speziell in Rauchgasen von Abfallverbrennungsanlagen, beschrieben. In diesem Verfahren werden eine Verdünnungssonde mit kritischer Düse und ein Katalysator aus borhaltigem Material eingesetzt. In Abgasen von Abfallverbrennungsanlagen ist bedingt durch die Inhomogenität des Abfalls mit kurzfristig hohen Kohlenmonoxidkonzentrationen zu rechnen. Diese CO-Spitzen verursachen relevante Fehlmessungen. Es wurde in der Praxis festgestellt, dass sich verwendete Katalysatoroberflächen trotz Verdünnung des Messgases durch weitere im Messgas vorhandene Komponenten belegen und der Katalysator bereits nach wenigen Wochen inaktiv wird. Dieses Verfahren zur Bestimmung von Quecksilber ist zur konzentrationsabhängigen Steuerung von Abscheidestufen nicht geeignet, da Fehlmessungen zur Initiierung bzw. Nichtinitiierung der Regelung führen können und eine Betriebssicherheit der Vorrichtung nicht gewährleistet ist.

DE 3838193 A1 beschreibt ein Verfahren und Messgerät zur kontinuierlichen Messung von Quecksilber und anderen Schwermetallen in Abgasen insbesondere von Müllverbrennungsanlagen. Als Reduktionsmittel wird Natriumborhydrid verwendet.

Diese Messverfahren erlauben jedoch lediglich eine Ermittlung der Quecksilberkonzentration. Die Abscheidung von Quecksilber lässt sich damit nicht beeinflussen.

Aufgabe der vorliegenden Erfindung ist es demzufolge, ein Verfahren zur Abscheidung von Quecksilber aus dem Abgasstrom so auszugestalten, dass eine zuverlässige Regelung der Reinigungsleistung der für die Reinigung des Abgasstroms verwendeten Reinigungsstufen möglich wird.

Bei dem erfindungsgemäßen Verfahren ist vorgesehen, dass ein Messgasstrom aus dem zu reinigenden Abgasstrom abgezweigt wird, dass in dem Messgasstrom eine Quecksilber-Konzentration gemessen wird und bei Überschreiten eines vorgebbaren Konzentrationswertes die Zudosierung der geeigneten Hilfsstoffe in den Abgasstrom und/oder die Aktivierung von temporären Reinigungsstufen erfolgt.

Vorzugsweise wird ein Messgasstrom aus einem noch nicht gereinigten Abgasstrom entnommen, in einem vorgebbaren Verhältnis mit einem geeigneten reduktiv wirkenden Gas oder Gasgemisch vermischt und die im Messgas enthaltenen Quecksilberverbindungen zu metallisches Quecksilber reduziert und anschließend einer Messvorrichtung zugeführt, mittels derer eine Bestimmung der Quecksilberkonzentration erfolgt.

Durch eine Vermischung des aus dem noch nicht gereinigten Abgasstroms entnommenen Messgasstroms mit einem geeigneten reduktiven Medium wie beispielsweise gereinigter Luft mit Kohlenmonoxid oder reinem Kohlenmonoxid wird das ionische Quecksilber durch die Anwesenheit von überschüssigem Kohlenmonoxid zu metallisches Quecksilber im feuchten Messgas reduziert und die Konzentration der weiteren Schadstoffkomponenten ebenfalls verringert, so dass eine dadurch verursachte Beeinträchtigung oder Verfälschung der Messungen der Quecksilberkonzentration weitgehend vermieden werden kann. Auf die Verwendung von in handelsüblichen Messgeräten für die Emissionsmessung eingebauten Reduktionseinheiten wie Katalysatoren oder nasschemische Reaktoren kann bei dieser Konstellation verzichtet werden. Gegebenenfalls ist die Anwendung eines handelsüblichen Alkaliabsorbers zur Separierung saurer Gase wie Chlorwasserstoff und Schwefeldioxid vorzusehen, insbesondere bei hohen Konzentrationen der sauren Gase.

Damit wird erstmals eine praxistaugliche Bestimmung der Quecksilberkonzentration bereits im Rohgasbereich ermöglicht.

In Abhängigkeit der ermittelten Quecksilberkonzentration des noch ungereinigten Abgasstroms kann dann in einfacher Weise über ein Steuerungsprogramm wie z. B. einer SPS 7, welches mit der SPS/PLS der Verbrennungsanlage verbunden ist, die Reinigungsleistung der nachfolgenden Reinigungsstufen vorgegeben, beziehungsweise angepasst werden. Die Verwendung handelsüblicher und einfach anpassbarer Messgeräte zur Bestimmung der Quecksilberkonzentration bzw. Steuerung der PLS erlaubt eine kostengünstige Umsetzung der Vorrichtung zur konzentrationsabhängigen Regelung der Reinigungsleistung der Abscheidestufen.

Es wurde festgestellt, dass eine hohe Konzentration von Kohlenmonoxid im Messgasstrom zu einer Verfälschung der Messwerte bei der Bestimmung der Quecksilberkonzentration führen kann. Dabei führen kurzzeitige hohe Konzentrationsschwankungen von Kohlenmonoxid, hervorgerufen durch wechselnde Verbrennungsbedingungen, oftmals zu einer Freisetzung von beispielsweise an den Wandungen der beheizten Messgasleitungen abgelagertem ionischen Quecksilber und damit zu einem vermeintlichen Anstieg der Quecksilberkonzentration in dem Messgasstrom.

Um eine nachteilige Beeinflussung durch zeitweilig hohe Kohlenmonoxidkonzentrationen zu verringern oder sogar vollständig auszuschließen ist vorgesehen, dass dem Messgasstrom eine ausreichende Menge von Kohlenmonoxid zugeführt wird. Auf diese Weise wird unabhängig von einer Kohlenmonoxidkonzentration im Abgasstrom eine Ablagerung von ionischem Quecksilber vermieden. Gleichzeitig fungiert das hinzugefügte Kohlenmonoxid als reduzierend wirkendes Gas. Durch eine kontinuierliche Zuführung von Kohlenmonoxid in wird eine Kohlenmonoxidkonzentration in dem Messgasstrom erhöht und es erfolgt eine überwiegend vollständige bzw. vollständige Reduktion von ionischem Quecksilber zu metallischem Quecksilber in dem Messgasstrom.

Die Regelung der Gasflüsse kann in einfacher Weise über Massflowregler bzw. einer kritischen Düse erfolgen. Die Zuführung von kohlenmonoxidhaltigem Gas sollte zweckmäßigerweise nahe an die Entnahmesvndeöffnung im Abgas (nach Partikelfilter) angeordnet sein.

Es ist aus der Praxis bekannt, dass gasförmiges ionisches Quecksilber, z.B. Quecksilber-II-Chlorid, welches überwiegend im Rohgas einer Abfallverbrennungsanlage vorliegt, bei niedrigen Temperaturen kondensieren und sich an Oberflächen niederschlagen und damit aus dem Abgasstrom, bzw. dem Messgasstrom abscheiden kann. In gleicher Weise kann abgelagertes ionisches Quecksilber bei hohen Temperaturen wieder desorbiert und in den Messgasstrom wieder aufgenommen werden. Der Einfluss der Umgebungstemperatur auf die Quecksilberkonzentration des Messgasstroms ist oftmals erheblich und kann dazu führen, dass die Bestimmung der gesamten Quecksilberkonzentration im Messgasstrom durch die Adsorption, beziehungsweise Desorption des gasförmigen ionischen Quecksilbers entlang des Förderwegs des Messgasstroms verfälscht wird.

Um eine derartige Beeinträchtigung der Gesamtquecksilber-Konzentrationsbestimmung zu vermindern, beziehungsweise vollständig auszuschließen ist vorgesehen, dass der Messgasstrom durch beheizbare Zuführungsleitungen der Messvorrichtung zugeführt wird. Die Zuführungsleitungen werden zweckmäßigerweise auf eine an die Temperatur des Messgasstroms angepasste Temperatur aufgeheizt, so dass der Messgasstrom keinen Temperaturschwankungen unterworfen wird und mit dem Messgasstrom in Kontakt kommende Oberflächen der Zuführungsleitungen einschließlich der Verbindungselemente, Gasaufbereitungsvorrichtungen etc. der Zuführungsleitungen eine einheitliche, gleichbleibende Temperatur aufweisen. Dabei ist eine Temperatur von etwa 453 Kelvin vorteilhaft, da bei dieser Temperatur die Kondensation und Adsorption von Quecksilber und Quecksilberverbindungen weitgehend verhindert werden kann und der Messgasstrom über weite Strecken während der Reinigung auf dieser Temperatur gehalten wird.

Um die Zuverlässigkeit der Bestimmung der Quecksilber-Konzentration im Rohgasbereich als unabdingbare Voraussetzung der Betriebssicherheit der Steuerung der Reinigungsleistung der Abscheidestufen zu gewährleisten ist vorgesehen, dass in zeitlich vorgebbaren Abständen ein Testgasstrom der Messvorrichtung zugeführt wird. Auf diese Weise kann beispielsweise täglich automatisiert überprüft werden, ob die Messvorrichtung ordnungsgemäß funktioniert und Messwertverfälschungen, wie sie beispielsweise durch nicht berücksichtigte Temperaturgradienten, eine nicht oder nur teilweise funktionierende Reduktion zu metallischem Quecksilber, ein nicht zutreffendes Vermischungsverhältnis, Querempfindlichkeiten z. B bei Anwesenheit von SO₂ in Konzentrationen über 1000 mg/Nm³ oder physikalische Störungen während der Bestimmung der Quecksilber-konzentration verursacht werden können.

Vorzugsweise ist dabei vorgesehen, dass der Testgasstrom weitgehend durch dieselben Zuführungsleitungen wie der Messgasstrom der Messvorrichtung zugeführt wird. Zweckmäßigerweise wird der Testgasstrom auch in dem gleichen Verhältnis wie der Messgasstrom mit dem CO-Gas vermischt Um Querempfindlichkeiten bei der Messung zu berücksichtigen, werden dem Testgasstrom weitere Spurengase wie beispielsweise SO₂ über die Vermischungseinheit zudosiert. Auf diese Weise können Beeinträchtigungen sowohl durch die Zuführungsleitungen, Querempfindlichkeiten wie auch Fehler bei der erforderlichen Vermischung des Messgasstroms erfasst und berücksichtigt werden.

Untersuchungen haben ergeben, dass die Messvorrichtung während des Betriebs in einer Abfallverbrennungsanlage trotz sorgfältiger Konzeption und Wartung der Messvorrichtung und der Zuführungsleitungen innerhalb kurzer Zeiträume von beispielsweise Wochen oder sogar Tagen durch Ablagerungen und Verunreinigungen beeinträchtigt wird, so dass eine Verfälschung der Messwerte auftreten kann. Eine regelmäßige Überprüfung der Messvorrichtung sowie der Zuführungsleitungen mittels des Testgasstroms erscheint deshalb nicht nur wünschenswert, sondern geboten.

Im Falle einer Verdünnung des Testgasstroms ist es erforderlich, dass der Testgasstrom eine Quecksilberkonzentration aufweist, die mit den üblicherweise auftretenden Quecksilberkonzentrationen des noch nicht gereinigten Abgasstroms übereinstimmt.

Um mit einfachen Mitteln möglichst zuverlässig einen Testgasstrom mit einer genau bekannten, gleichbleibenden Quecksilberkonzentration bereitstellen zu können ist vorgesehen, dass der Testgasstrom durch Kapillardiffusion von gasförmigem Quecksilber-11-Chlorid, anderen geeigneten Quecksilbersalzen bzw. -oxiden oder metallisches Quecksilber in einen vorgegebenen Trägergasstrom durch Erhitzen erzeugt wird. Der beispielsweise in einem beheizbaren Vorratsbehälter erzeugte Quecksilbergasstrom diffundiert mit im Wesentlichen gleichbleibender Rate durch eine Kapillare in den Trägergasstrom. Als Trägergasstrom wird zweckmäßigerweise ein ebenfalls vorgeheizter Gasstrom, beispielsweise ein getrockneter ,stickstoffhaltiger Gasstrom mit gegebenenfalls weiteren Zusätzen wie beispielsweise SO₂ und CO verwendet. Eine Konzentration von 2000 mg/Nm³ SO₂ entspricht oftmals den realen Verhältnissen des Rohgases und hat sich in der Praxis bewährt.

Untersuchungen der Anmelderin haben ergeben, dass das reduzierend wirkende Gas wie z. B. Kohlenmonoxid oder Luft mit Kohlenmonoxid in einem hohen Überschuss in Bezug auf ionisches Quecksilber und eine entsprechende Feuchte im Messgas vorhanden sein muss.

Um eine vorteilhafte Regelung der nachfolgenden Reinigung zu ermöglichen ist vorgesehen, dass die Messvorrichtung eine Einrichtung zur Bestimmung der Gesamtkonzentration von Quecksilber und eine Einrichtung zur Bestimmung der Konzentration von metallischem Quecksilber aufweist. Da metallisches Quecksilber einerseits und ionisches Quecksilber andererseits nur mit unterschiedlichen Reinigungsverfahren effizient aus dem Abgasstrom entfernt werden kann, ermöglicht die getrennte Bestimmung der jeweiligen Quecksilberkonzentration in dem Messgasstrom eine verbesserte Anpassung der jeweiligen Reinigungsleistung. Auf diese Weise können eine verminderte Umweltbelastung und ein geringerer Kostenaufwand gegenüber einer ansonsten üblichen Annahme eines festen Verhältnisses der Konzentration von metallischem Quecksilber zu ionischem Quecksilber, beziehungsweise der Gesamtkonzentration von Quecksilber erzielt werden.

Zur Bestimmung der Konzentration von metallischem Quecksilber wird aus dem noch nicht gereinigten Abgasstrom ein Messgasstrom entnommen und vor der Zuführung zu einer Messvorrichtung von störenden Komponenten befreit. Dies wird zweckmäßigerweise dadurch erreicht, dass der Messgasstrom nacheinander durch einen Alkaliabsorber zur Entfernung von sauren Gasen und insbesondere von Schwefeldioxid und Chlorwasserstoff und durch einen Messgaskühler zur Entfernung von Feuchte und Salzfrachten geführt wird, bevor der Messgasstrom der Messvorrichtung zugeführt wird. Anstatt der Separierung von störenden Komponenten können mittels chemometrischer Auswertung der Spektren im Falle der Anwendung spektroskopischer Messverfahren die Störeinflüsse minimiert bzw. vollständig eliminiert werden.

Um die oftmals empfindlichen Messvorrichtungen vor einer Beeinträchtigung oder Beschädigung durch eine übermäßig hohe Quecksilberkonzentration zu schützen ist vorgesehen, dass die Zuführung des Messgasstroms an die Messvorrichtung unterbrochen wird, sobald die ermittelte Quecksilberkonzentration einen vorgegebenen Schwellenwert übersteigt.

Erfindungsgemäß ist demzufolge vorgesehen, dass eine Bestimmung der Quecksilberkonzentration des noch nicht gereinigten Abgasstroms mit einem der vorangehend beschriebenen Verfahren vorgenommen und in Abhängigkeit von der jeweils aktuell ermittelten Quecksilberkonzentration eine Zudosierung der geeigneten Hilfsstoffe erfolgt. In Abhängigkeit von der ermittelten Konzentration von ionischem Quecksilber (Gesamtquecksilber - metallischer Anteil= ionischer Anteil) kann beispielsweise die Zudosierung einer Natriumsulfidlauge in den Nasswäscher gesteuert werden. Wird ein vorgegebener Schwellenwert der Konzentration von ionischem Quecksilber überschritten, so kann gegebenenfalls zusätzlich ein geeignetes Fällungsmittel zur Ausfällung von ionischen Quecksilberverbindungen zugegeben werden. Untersuchungen haben ergeben, dass beispielsweise bei einem Massenstrom von ca. 3 kg/h an ionischem Quecksilber ein kritischer Wert für die weitere Erhöhung der Dosiermenge von Natriumsulfidlauge erreicht ist, da ansonsten die Gefahr der Überschreitung des Emissionsgrenzwertes für Schwefelwasserstoff besteht.

In Abhängigkeit der Konzentration von metallischem und Gesamtquecksilber kann beispielsweise die Zugabe von mit Schwefelsäure dotierter Aktivkohle, und/oder von einer alkalischen Natriumdithionitlösung gesteuert werden. Auf diese Weise werden adäquate Mengen von Fällungschemikalien und Adsorbentien an die bei der Verbrennung des Abfalls entstehende Quecksilberbelastung angepasst und nur in dem Maße zudosiert, wie es erforderlich ist.

Die Erfindung betrifft auch eine Vorrichtung zur Abscheidung von Quecksilber aus Abgasen von Verbrennungsanlagen, insbesondere von Abfallverbrennungsanlagen, wobei einem Abgasstrom geeignete Hilfsstoffe zur Abscheidung des Quecksilbers zugeführt werden, mit einer Messgasvorrichtung zur Bestimmung der Quecksilber-Konzentration, wobei die Messgasvorrichtung über Zuführungsleitungen mit dem Abgasstrom im Rohgasbereich verbindbar ist, mit einer zwischen dem Rohgasbereich und der Messgasvorrichtung angeordneten Vermischungsvorrichtung und mit Mitteln zur Steuerung der Zudosierung der Hilfsstoffe zu dem zu reinigenden Abgasstrom.

Erfindungsgemäß ist vorgesehen, dass die Messvorrichtung über zuführungsleitungen mit einem Rohgasbereich eines noch nicht gereinigten Abgasstroms verbindbar ist und dass zwischen dem Rohgasbereich und der Messvorrichtung eine Vermischungsvorrichtung angeordnet ist. Aus dem noch nicht gereinigten Abgasstrom kann dann ein Messgasstrom abgezweigt und nach Abtrennung staubhaltiger Bestandteile mittels Filtration über die Zuführungsleitungen der Messvorrichtung zugeführt werden. Bevor der Messgasstrom der Messvorrichtung zugeführt wird, wird der Messgasstrom mit der Vermischungsvorrichtung in einem vorgebbaren Verhältnis mit einem reduktiven Medium vermischt. Ein geeignetes reduktiv wirkendes Medium ist beispielsweise gereinigte und vorgewärmte Luft im Gemisch mit Kohlenmonoxid oder reines CO-Gas, wobei die Temperatur des Mediums zweckmäßiger Weise an die Temperatur des Messgasstroms angepasst ist. Auf diese Weise kann auch mit bereits bekannten und handelsüblich erhältlichen Messvorrichtungen eine zuverlässige Bestimmung der Quecksilberbelastung eines Abgasstroms im Rohgasbereich als Leitwert für die Regelung der Reinigungsleistung von Reinigungsstufen vorgenommen werden.

Bei der Messvorrichtung handelt es sich zweckmäßigerweise um eine Messvorrichtung, die zur Bestimmung der Gesamtquecksilberkonzentration geeignet ist. Für viele Anwendungen ist eine spektroskopisch ermittelnde Messvorrichtung geeignet und vorteilhaft anwendbar. Eine Reduktionseinheit die üblicherweise in handelsüblichen Messvorrichtungen enthalten ist, kann aufgrund der Zuführung von kohlenmonoxidhaltigem Gas in das feuchte Messgas verzichtet werden.

Speziell besteht die Vorrichtung aus Messeinrichtungen zur Messung von Gesamt- und metallischem Quecksilber, einem Testgasgenerator zur Erzeugung von quecksilberhaltigen Testgasen zur Sicherstellung einer störungsfreien Betriebsweise und einem Steuermodul zur konzentrationsabhängigen Regulierung der Reinigungsleistung von Abscheidestufen für Quecksilber.

Vorzugsweise ist vorgesehen, dass die Zuführungsleitungen mindestens teilweise beheizbar sind. Der zu reinigende Abgasstrom und damit auch der daraus abgezweigte Messgasstrom weisen üblicherweise eine Temperatur von etwa 453 Kelvin auf. Eine unerwünschte Abscheidung oder Anreicherung des in dem Messgasstrom enthaltenen ionischen Quecksilbers kann durch eine Aufheizung der Zuführungsleitungen auf ebenfalls 453 Kelvin weitgehend unterbunden werden.

Um eine in zeitlichen Abständen erfolgende Überprüfung der Messvorrichtung zu ermöglichen ist vorgesehen, dass zwischen dem Rohgasbereich und der Vermischungsvorrichtung eine oder mehrere Vorrichtungen zur Erzeugung eines Testgasstroms angeordnet ist.

Gemäß einer besonders vorteilhaften Ausgestaltung des Erfindergedankens ist vorgesehen, dass die Vorrichtung zur Erzeugung eines Testgasstroms einen beheizbaren, austauschbaren Vorratsbehälter mit festverbundenem Kapillarenaufsatz hat, wobei die Kapillare von einem Trägergasstrom umströmt wird. In den Vorratsbehälter kann eine bei Raumtemperatur feste Quecksilberverbindung wie beispielsweise Quecksilber-II-Chlorid oder metallisches Quecksilber über die Kapillarenöffnung (z.B. dᵢ=5 mm) eingebracht, kontrolliert erhitzt und verdampft werden. Das in dem Vorratsbehälter erzeugte quecksilberhaltige Gas diffundiert mit einer gleichbleibenden Diffusionsrate aus der Kapillare heraus und wird von dem Trägergasstrom aufgenommen und mitgeführt. Die Diffusionsrate ist dabei im Wesentlichen von der Temperatur und der Geometrie der Kapillare abhängig, die äußerst präzise vorgegeben werden können.

Untersuchungen haben ergeben, dass die nachfolgend genannten Konstruktionsmerkmale der Vorrichtung zur Erzeugung eines Testgasstroms vorteilhaft bei der Abfallverbrennung berücksichtigt werden können:
Das Verhältnis von Kapillarquerschnitt relativ zur Länge der Kapillare sollte geringer als 0,3 sein, der Durchmesser der Kapillare sollte geringer als 20 mm sein und der Trägergasstrom insbesondere bei kleineren Kapillardurchmessern bis 5 mm eine Flowrate von 10-50 1/h aufweisen.

Um unterschiedliche Quecksilberkonzentrationen des Testgasstroms bei gleichbleibender Temperatur und unverändertem Trägergasstrom zu ermöglichen ist vorgesehen, dass ein Vorratsgefäß mit unterschiedlichem Kapillaraufsatz verwendet werden kann. Durch die Verwendung unterschiedlicher Kapillaren können in einfacher Weise und kurzfristig Testgasströme mit beispielsweise unterschiedlichen Quecksilber-II-Chloridkonzentrationen erzeugt werden.

Es ist auch denkbar, dass mehrere Vorrichtungen zur Erzeugung eines Testgasstroms mit jeweils unterschiedlichen Kapillaraufsätzen gleichzeitig gehalten und abwechselnd zur Überprüfung der Messvorrichtung verwendet werden.

Zur Erzeugung von Testgasen mit extrem unterschiedlichen Quecksilberkonzentrationen, wie sie zur Bereitstellung von Testgasen für die Gesamt- und metallische Quecksilbermessung erforderlich sind, kann die gleiche Vorrichtung verwendet werden, da mit dieser Methode üblicherweise Quecksilberkonzentrationen von 1 - 100 mg/m³ erzeugt werden können. Besonders vorteilhaft ist es, einen angefeuchteten CO-Gasstrom als Verdünnungsgas zur Einstellung der erforderlichen Testgaskonzentration bei Anwendung von Quecksilbersalzen oder -oxiden zu verwenden, da hierdurch eine Reduktion zu metallischem Quecksilber erfolgt und auf die üblicherweise in kommerziell erhältlichen Testgasgeneratoren zur Erzeugung sehr niedriger Konzentrationen im µg/Nm³ Bereich verwendeten Reduktionsverfahren verzichtet werden kann.

Nachfolgend werden Ausführungsbeispiele des Erfindungsgedankens näher erläutert, die in der Zeichnung dargestellt sind. Es zeigt:
Fig. 1 eine schematische Darstellung eines Verfahrens zur Reinigung eines quecksilberhaltigen Abgasstroms, wobei die Reinigungsleistung in Abhängigkeit von der ermittelten Quecksilberbelastung des Abgasstroms gesteuert werden kann,
Fig. 2 eine schematische Darstellung einer Messvorrichtung, der über Zuführungsleitungen ein Messgasstrom oder ein Testgasstrom zugeführt werden kann,
Fig. 3 eine schematische Darstellung einer anderen Messvorrichtung, der über Zuführungsleitungen ein Messgasstrom oder ein Testgasstrom zugeführt werden kann, und
Fig. 4 eine schematische Darstellung einer Vorrichtung zur Erzeugung eines Testgasstroms.

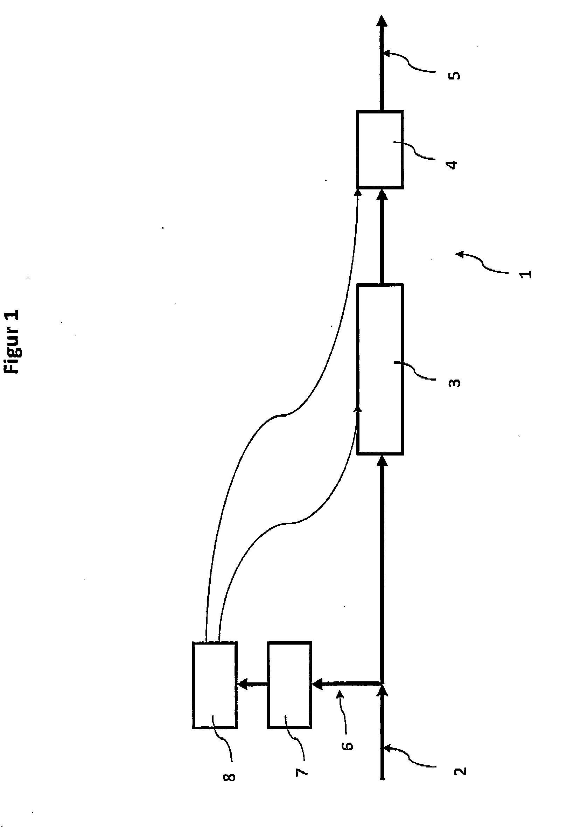

In Figur 1 wird lediglich beispielhaft und schematisch eine regelbare Reinigungsvorrichtung 1 zur Reinigung eines quecksilberhaltigen Abgasstroms 2 dargestellt. Der noch nicht gereinigte Abgasstrom 2 wird zu diesem Zweck durch lediglich symbolisch angedeutete Reinigungsstufen 3 und 4 der Reinigungsvorrichtung 1 hindurchgeführt und dabei gereinigt. Aus der Reinigungsvorrichtung 1 tritt ein gereinigter Abgasstrom 5 aus, der in die Atmosphäre abgegeben werden kann.

In den Reinigungsstufen 3 und 4 können metallisches Quecksilber sowie quecksilberhaltige Verbindungen aus dem zu reinigenden Abgasstrom 2 entfernt werden. Zu diesem Zweck werden dem zu reinigenden Abgasstrom 2 Hilfsstoffe wie beispielsweise Fällungschemikalien und Adsorbentien zudosiert, die gezielt eine Ausfällung und Abscheidung von Quecksilber, bzw. von quecksilberhaltigen Verbindungen bewirken.

Um die Zudosierung der Hilfsstoffe in Abhängigkeit von der tatsächlich gegebenen Quecksilberbelastung des Abgasstroms 2 steuern, bzw. regeln zu können, wird vor dem Eintritt in die Reinigungsstufen 3 und 4 ein Messgasstrom 6 von dem zu reinigenden Abgasstrom 2 abgezweigt. Der Messgasstrom 6 wird anschließend in einer Vermischungsvorrichtung 7 in einem vorgegebenen Verhältnis mit einem geeigneten reduktiv wirkenden Medium, dass Kohlenmonoxid im Überschuss enthält vermischt, so dass ionisches Quecksilber vollständig zu metallisches Quecksilber reduziert wird. Bei hohen Konzentrationen an sauren Gasen im Messgasstrom ist zu empfehlen, diese durch einen geeigneten Alkaliabsorber zu entfernen.

Der mit CO-Gas vermischte Messgasstrom 6 wird anschließend einer Messvorrichtung 8 zugeführt. In der Messvorrichtung 8 wird eine Bestimmung der Quecksilberkonzentration vorgenommen. In Abhängigkeit von der ermittelten Quecksilberkonzentration kann dann die Zudosierung der Hilfsstoffe in den Reinigungsstufen 3 und 4, bzw. deren Reinigungsleistung gesteuert werden.

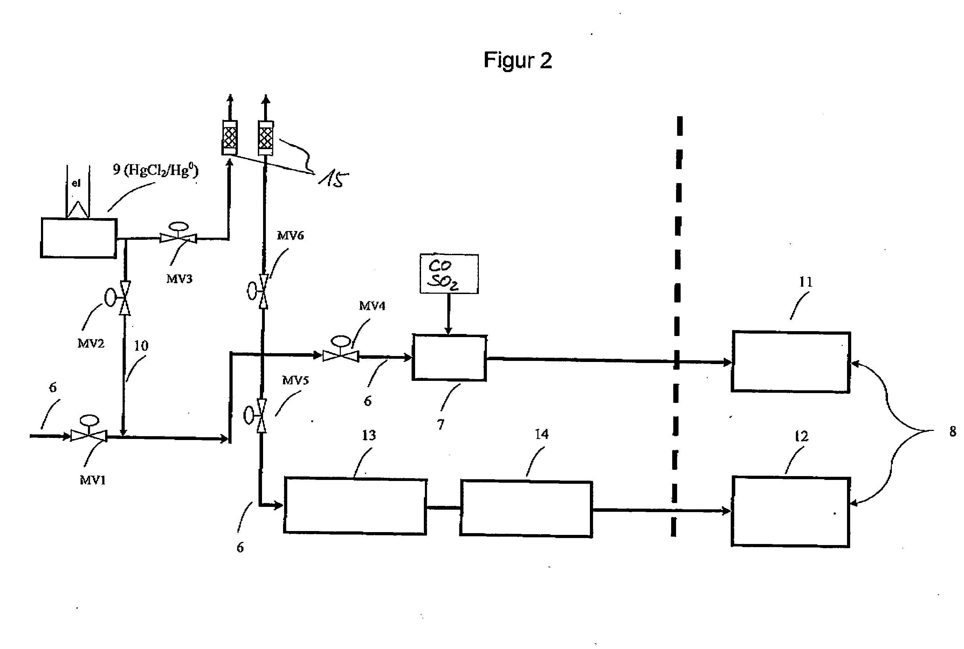

In Figur 2 wird anhand einer ebenfalls schematischen Darstellung die bereits in Figur 1 gezeigte Messvorrichtung 8 zusammen mit der Vermischungsvorrichtung 7 und einer Vorrichtung 9 zur Erzeugung eines Testgasstroms 10 abgebildet. Bei einem geöffneten Ventil MV4 kann entweder ein dem zu reinigenden Abgasstrom 2 abgezweigter Messgasstrom 6 oder ein mit der Vorrichtung 9 zur Erzeugung eines Testgasstroms erzeugter Testgasstrom 10 der Vermischungsvorrichtung 7 und anschließend der Messvorrichtung 8 zugeführt werden. Im Falle der Zuführung eines Testgasstroms 10 kann über die Vermischungseinheit 7 beispielsweise zusätzlich SO₂-Gas zugeführt werden.

Die lediglich beispielhaft dargestellte Messvorrichtung weist zu diesem Zweck ein handelsübliches Messgerät 11 zur Bestimmung der Gesamtkonzentration von Quecksilber in dem Messgasstrom auf. Um eine unerwünschte Beeinträchtigung der mit dem Messgerät 11 durchgeführten Messungen zu vermeiden sind die Zuführungsleitungen des Messgasstromes 6 bzw. Testgasstromes 10 beheizbar und auf die Temperatur des Messgasstroms 6 vorgeheizt.

Die Messvorrichtung kann ein weiteres Messgerät 12 zur Bestimmung von gasförmigem metallischem Quecksilber aufweisen. Geeignete handelsüblich erhältliche Messgeräte 12 erfordern allerdings einen vorgeschalteten Messgaskühler 13 zur Abscheidung von Salzen und Feuchte (beispielsweise Ammoniumsalzen)und eine Trennstufe 14 (beispielsweise ein Alkalifestbettadsorber). Die zu dem Messgerät 12 führenden Zuführungsleitungen des Messgasstromes 6 bzw. Testgasstromes 10 müssen demzufolge nicht beheizbar sein. Das Messgerät 12 wird bei geöffnetem Ventil MV5 verwendet.

Nicht benötigte Mengen des Testgasstroms 10, bzw. des Messgasstroms 6 können über Ventile MV3 und MV6 einer Reinigungseinrichtung 15 zugeführt und anschließend in die Umgebung abgegeben werden.

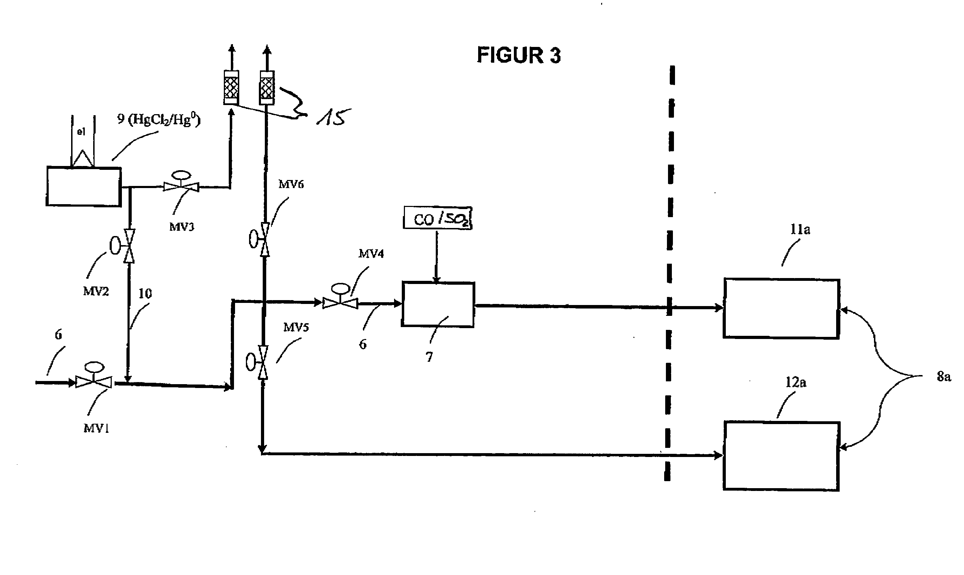

In Figur 3 ist beispielhaft in einer schematischen Darstellung die Anwendung eines spektrometrischen Messverfahrens mit zwei auf den Konzentrationsbereich angepassten Messgeräten, bzw. Transmissionszellen 11a und 12a zur simultanen Messung von Gesamtquecksilber und metallischem Quecksilber mit problemangepasster Schichtdicke der Transmissionszellen 11a und 12a und einer Multiplexschaltung auf ein Detektorsystem dargestellt, dass für einen Temperaturbereich bis beispielsweise 553 K ausgelegt ist und eine chemometrische Auswertung der Spektren ermöglicht. Bei dieser Anwendung ist für die Bestimmung des Gesamtgehaltes an Quecksilber ebenfalls eine Zuführung von CO-haltigem Gasgemisch bzw. reinem CO-Gas im Überschuss erforderlich, um als zu messende Komponente metallisches Quecksilber vorliegen zu haben. Für die Bestimmung des metallischen Quecksilbers kann auf den vorgeschalteten Messgaskühler 13 und der Trennstufe 14 verzichtet werden. Allerdings ist dann eine Beheizung der Messgasleitungen analog dem Messgasstrom 6 auf ca. 453 K notwendig. Durch das Öffnen, bzw. Schließen der Ventile MV1 und MV2 können bei geöffneten Ventilen MV4 und MV5 der Messgasstrom 6, bzw. der Testgasstrom 10 selektiv den Transmissionszellen 11a und 12a zugeführt werden.

Durch die getrennte Messung der Gesamtkonzentration von Quecksilber mit dem Messgerät 11 sowie die gesonderte Erfassung der Konzentration von metallischem Quecksilber mit dem Messgerät 12 bzw. der simultanen Messung mittels der Transmissionszellen 11a und 12a können die zur Abscheidung von ionischem Quecksilber einerseits und metallischem Quecksilber andererseits erforderlichen Hilfsstoffe und Reinigungsverfahren in geeigneter Weise in den Reinigungsstufen 3 und 4 zudosiert, bzw. gesteuert werden.

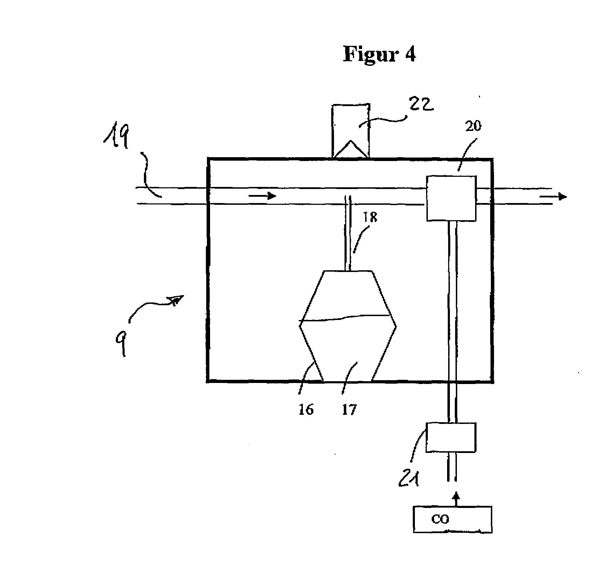

In Figur 4 ist lediglich beispielhaft und schematisch eine Ausgestaltung der Vorrichtung 9 zur Erzeugung eines Testgasstroms 10 für Quecksilber dargestellt. Die auf ± 0,1 K genau beheizbare Vorrichtung 9 weist einen Vorratsbehälter 16 auf, in welchem ein bei Raumtemperatur festes Quecksilber-II-Chlorid, anderes geeignetes quecksilberhaltiges Salz bzw. -oxid oder metallisches Quecksilber 17 erhitzt und verdampft werden kann. Mit dem Vorratsbehälter 16 ist in einfacher Weise auswechselbar eine Kapillare 18 verbundenen. Der Vorratsbehälter 16 kann über die Kapillare 18 befüllt werden. Durch die fest verbundene Kapillare 18 kann das in dem Vorratsbehälter beispielsweise verdampfte Quecksilber-II-Chlorid 17 diffundieren und aus dem Vorratsbehälter 16 austreten.

Ein Trägergasstrom 19 transportiert das diffundierte quecksilberhaltige Testgas 10 in eine Misch- und Redukionseinheit 20, in der der Testgasstrom 10 auf die gewünschte Konzentration verdünnt und durch Zuleitung eines über einen Befeuchter 21 befeuchteten CO-Gasstromes zu metallisches Quecksilber reduziert wird. Die Zuleitungen für Träger- und befeuchtetem CO-Gas, der Vorratsbehälter 16 sowie die Misch- und Redukionseinheit 20 sind von einer lediglich schematisch angedeuteten Heizvorrichtung 22 umgeben, mit welcher der Vorratsbehälter 16 und die Misch- und Redukionseinheit 20 einschließlich des darin erzeugten Testgasstroms 10 auf eine vorgegebene Temperatur gebracht, bzw. dort gehalten werden können.

## Patentansprüche

1. Verfahren zur Abscheidung von Quecksilber aus Abgasen von Verbrennungsanlagen, insbesondere von Abfallverbrennungsanlagen, wobei einem Abgasstrom geeignete Hilfsstoffe zur Abscheidung des Quecksilbers zugeführt werden, **dadurch gekennzeichnet, dass** ein Messgasstrom aus (6) dem zu reinigenden Abgasstrom abgezweigt wird, dass in dem Messgasstrom (6) eine Quecksilber-Konzentration gemessen wird und bei Überschreiten eines vorgebbaren Konzentrationswertes die Zudosierung der geeigneten Hilfsstoffe in den Abgasstrom und/oder die Aktivierung von temporären Reinigungsstufen erfolgt.

2. Verfahren nach Anspruch 1, **dadurch gekennzeichnet, dass** der Messgasstrom (6) zur Bestimmung der Quecksilberkonzentration in einem vorgebbaren Verhältnis mit einem reduktiven Medium, welches CO-Gas im Überschuss enthält, vermischt wird und der vermischte Messgasstrom (6) einer Messvorrichtung (8, 8a) zugeführt wird.

3. Verfahren nach Anspruch 1 oder 2, **dadurch gekennzeichnet, dass** dem Messgasstrom (6) eine ausreichende Menge von Kohlenmonoxid zugeführt wird.

4. Verfahren nach einem der vorangehenden Ansprüche, **dadurch gekennzeichnet, dass** eine Kohlenmonoxidkonzentration in den Messgasstrom (6) kontinuierlich durch Zuführung von Kohlenmonoxid erhöht wird und eine überwiegend vollständige bzw. vollständige Reduktion von ionischem Quecksilber zu metallischem Quecksilber in dem Messgasstrom (6) erfolgt.

5. Verfahren nach einem der vorangehenden Ansprüche, dass bei Anwendung spektroskopischer Verfahren die Auswertung der Spektren mittels chemometrischer Methoden erfolgt und eine Kompensation von Störkomponenten vorgenommen wird.

6. Verfahren nach einem der vorangehenden Ansprüche, **dadurch gekennzeichnet, dass** in zeitlich vorgebbaren Abständen ein Testgasstrom (10) der Messvorrichtung (8) bzw. (8a)zugeführt wird.

7. Verfahren nach einem der vorangehenden Ansprüche,
**dadurch gekennzeichnet, dass** der Testgasstrom (10) weitgehend durch dieselben Zuführungsleitungen wie der Messgasstrom (6) der Messvorrichtung (8) bzw. (8a) zugeführt wird.

8. Verfahren nach Anspruch 8 oder Anspruch 9, **dadurch gekennzeichnet, dass** der Testgasstrom (10) durch Kapillardiffusion eines vorgegebenen Quecksilber-II-Chloridsalzes anderer geeigneter quecksilberhaltiger Salze bzw. -oxide oder metallisches Quecksilber in einen vorgegebenen Trägergasstrom (19) erzeugt wird und durch Zuführung von befeuchtetem CO-Gas zu einem metallischen Quecksilbertestgasstrom reduziert wird.

9. Verfahren nach einem der vorangehenden Ansprüche, **dadurch gekennzeichnet, dass** der Messgasstrom (6) durch beheizbare Zuführungsleitungen des Messgasstromes (6) der Messvorrichtung (8) bzw. (8a)zugeführt wird.

10. Verfahren nach einem der vorangehenden Ansprüche, **dadurch gekennzeichnet, dass** der Testgasstrom (10) durch beheizbare Zuführungsleitungen des Testgasstromes (10) der Messvorrichtung (8) bzw. (8a) zugeführt wird

11. verfahren nach einem der vorangehenden Ansprüche, **dadurch gekennzeichnet, dass** die Messvorrichtung (8) eine Einrichtung (11) zur Bestimmung der Gesamtkonzentration von Quecksilber und eine Einrichtung (12) zur Bestimmung der Konzentration von metallischem Quecksilber aufweist.

12. Verfahren nach einem der Ansprüche 1 bis 10, **dadurch gekennzeichnet, dass** die Messvorrichtung (8a) Transmissionszellen (11a, 12a) zur simultanen Messung von Gesamtquecksilber und metallischem Quecksilber mit problemangepasster Schichtdicke der Transmissionszellen (11a, 12a) und einer Multiplexschaltung aufweist.

13. Verfahren nach einem der vorangehenden Ansprüche, **dadurch gekennzeichnet, dass** die Zuführung des Messgasstroms (6) an die Messgasvorrichtung (8) bzw. (8a) unterbrochen wird, sobald eine ermittelte Quecksilberkonzentration einen vorgegebenen Schwellenwert übersteigt.

14. Vorrichtung zur Abscheidung von Quecksilber aus Abgasen von Verbrennungsanlagen, insbesondere von Abfallverbrennungsanlagen, wobei einem Abgasstrom geeignete Hilfsstoffe zur Abscheidung des Quecksilbers zugeführt werden, mit einer Messgasvorrichtung (8) bzw. (8a)zur Bestimmung der Quecksilber-Konzentration, wobei die Messgasvorrichtung (8) bzw. (8a)über Zuführungsleitungen mit dem Abgasstrom (2) im Rohgasbereich verbindbar ist und mit Mitteln zur Steuerung der Zudosierung der Hilfsstoffe zu dem zu reinigenden Abgasstrom.

15. Vorrichtung nach Anspruch 14, **dadurch gekennzeichnet, dass** eine Vermischungsvorrichtung (7) zwischen dem Rohgasbereich und der Messgasvorrichtung (8) angeordnet ist.

16. Vorrichtung nach einem der Ansprüche 14 oder 15, **dadurch gekennzeichnet, dass** die Zuführungsleitungen mindestens teilweise beheizbar sind.

17. Vorrichtung nach einem der Ansprüche 14 bis 16, **dadurch gekennzeichnet, dass** zwischen dem Rohgasbereich und der Messvorrichtung (8, 8a) eine oder mehrere Vorrichtungen (9) zur Erzeugung eines Testgasstroms (10) angeordnet ist, bzw. sind.

18. Vorrichtung nach Anspruch 17, **dadurch gekennzeichnet, dass** die Vorrichtung (9) zur Erzeugung eines Testgasstroms (10) einen wechselbaren beheizbaren Vorratsbehälter (16) mit einer jeweils fest verbundenen Kapillare (18) aufweist, wobei die Kapillare (18) von einem Trägergasstrom (19) umströmt wird.
